(19) Europäisches Patentamt
European Patent Office
Office européen des brevets

(11) EP 1 964 930 A1

(12) EUROPEAN PATENT APPLICATION

(43) Date of publication:
03.09.2008 Bulletin 2008/36

(51) Int Cl.:
*C12Q 1/68* (2006.01)

(21) Application number: 07290251.3

(22) Date of filing: 28.02.2007

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK RS

(71) Applicant: INSTITUT NATIONAL DE LA SANTE ET DE LA RECHERCHE MEDICALE (INSERM)
75013 Paris (FR)

(72) Inventors:
• Minvielle, Stéphane
44620 La Montagne (FR)

• Avet-Loiseau, Hervé
44000 Nantes (FR)
• Magrangeas, Florence
44620 La Montagne (FR)
• Campion, Loïc
44300 Nantes (FR)

(74) Representative: Vialle-Presles, Marie José et al
Cabinet Orès
36, rue de ST Pétersbourg
F-75008 Paris (FR)

(54) **Molecular classifier for prognosis in multiple myeloma**

(57) The invention relates to a 15-gene molecular classifier consisting of the following genes: CNDP2; STMN1; AFG3L2; STK38; PARP1; CPSF6; LOC151162; C20orf100; FRY; FLJ21438; MGST1; ALDH2; CTSF; ATF4; FAM49A. The level of expression of these genes in bone marrow plasma cells of patients with multiple myeloma is a useful marker for evaluating their probability of survival.

Figure 3

**Description**

The invention relates to methods for prognosis in multiple myeloma.

Multiple myeloma is one of the most common forms of haematological malignancy. It occurs in increasing frequently with advancing age, with a median age at diagnosis of about 65 years.

It is associated with an uncontrolled clonal proliferation of B-lymphocyte-derived plasma cells within the bone marrow. These myeloma cells replace the normal bone marrow cells, resulting in an abnormal production of cytokines, and in a variety of pathological effects, including for instance anemia, hypercalcemia, immunodeficiency, lytic bone lesions, and renal failure. The presence of a monoclonal immunoglobulin is frequently observed in the serum and/or urine of multiple myeloma patients.

Current treatments for multiple myeloma patients include chemotherapy, preferably associated when possible with autologous stem cell transplantation (ASCT).

Typically, treatments for patients eligible for ASCT include (1) *Initial chemotherapy.* Patients are initially treated with a continuous intravenous infusion of 0.4 mg of vincristine per square meter of body surface area and 9 mg of doxorubicin per square meter over a 24 hour period for 4 days, with 40 mg of oral dexamethasone per day on days 1 through 4 (the VAD regimen). Three to four cycles of VAD are administered at 3-week intervals. (2) *Autologous stem cell collection.* After initial chemotherapy, patients under 66 years of age with a performance status below World Health Organization grade 3 and a serum creatinine level of less than 150 micromoles per liter undergo blood stem cell collection. (3) *Stem cell transplantation.* Double ASCT is performed. Melphalan alone is given before each ASCT (usually 200 mg per square meter).

Patients ineligible for transplantation because of age (> 65 years) or poor physical condition are treated with the regimen consisted of 12 courses at 6-week cycles of melphalan (0.25 mg/kg/j J1-J4) and prednisone (2 mg/kg/j Jl-J4) plus/minus thalidomide (< 400mg/day).

Survival of patients with multiple myeloma is highly heterogeneous from periods of few weeks to more than ten years.

This variability derives from heterogeneity in both tumor and host factors. It is thus important to identify factors associated with prognosis, in order to better predict disease outcome, and optimize patient treatment.

Several factors correlated with survival duration in multiple myeloma have been identified, including in particular serum levels of hemoglobin, calcium, creatinine, $\beta$2-microglobulin ($S\beta_2M$), albumin, C-reactive protein (CRP), platelets count, proliferative activity of bone marrow plasma cells, and deletion of chromosome arm 13q. Subsequently, various combinations of prognostic factors have been suggested for staging classification of myeloma patients. Recently, a staging system for multiple myeloma has been developed through an international collaboration between several teams (GREIPP et al., J Clin Oncol, 23, 3412-20, 2005). A combination of $S\beta_2M$ and serum albumin was retained as providing the simplest, most powerful and reproducible three-stage classification. This International Staging System (ISS) consists of the following stages: stage I, $S\beta_2M$ less than 3.5 mg/L plus serum albumin $\geq$3.5 g/dL (median survival, 62 months); stage II, neither stage I nor III (median survival, 44 months); and stage III, $S\beta_2M\geq$5.5 mg/L (median survival, 29 months).

The ISS is easy to use and provide useful prognostic groupings in a variety of situations. However it does not allow an accurate identification of higher risk patients. Thus, there remains a need to develop other staging systems that would further help to establish clinically relevant grouping of multiple myeloma patients.

The inventors hypothesized that gene expression profiling could improve the accuracy of staging. Starting from a microarray of 17134 EST cDNA clones representing 11250 unique genes, they have designed a molecular classifier based on a set of only 15 genes, which were highly predictive of survival when used together.

These 15 genes are listed in Table I below.

Table I

| Reference Sequence | Gene Description | Gene Symbol |
|---|---|---|
| NM_018235 | CNDP dipeptidase 2 (metallopeptidase M20 family) | CNDP2 |
| AK123488 | Stathmin 1/oncoprotein 18 | STMN1 |
| NM_006796 | AFG3 ATPase family gene 3-like 2 (yeast) | AFG3L2 |
| NM_007271 | Serine/threonine kinase 38 | STK38 |
| NM_001618 | Poly (ADP-ribose) polymerase family, member 1 | PARP1 |
| NM_007007 | Cleavage and polyadenylation specific factor 6, 68kDa | CPSF6 |
| BX647087 | Hypothetical protein LOC151162 | LOC151162 |
| NM_032883 | Chromosome 20 open reading frame 100 | C20orf100 |
| NM_023037 | Furry homolog (Drosophila) | FRY |
| AK024488 | Hypothetical protein FLJ21438 | FLJ21438 |

(continued)

| Reference Sequence | Gene Description | Gene Symbol |
|---|---|---|
| NM_145791 | Microsomal glutathione S-transferase 1 | MGST1 |
| NM_000690 | Aldehyde dehydrogenase 2 family (mitochondrial) | ALDH2 |
| NM_003793 | Cathepsin F | CTSF |
| NM_001675 | Activating transcription factor 4 (tax-responsive enhancer element B67) | ATF4 ATF4 |
| AK055334 | Family with sequence similarity 49, member A | FAM49A |

These 15 genes will be designated hereinafter by the gene symbols indicated in Table I, which are those approved by the HUGO Gene Nomenclature Committee (HGNC).

The invention thus relates to a method for evaluating the probability of survival at a predetermined date for a patient with multiple myeloma, wherein said method comprises measuring the level of expression of each of the following genes: CNDP2; STMN1; AFG3L2; STK38; PARP1; CPSF6; LOC151162; C20orf100; FRY; FLJ21438; MGST1; ALDH2; CTSF; ATF4; FAM49A, in a sample of bone marrow CD138+ plasma cells obtained from said patient.

According to an embodiment of the invention, the level of expression of each of said genes is compared with the mean level of expression of the same gene previously evaluated in bone marrow CD138+ plasma cells from a reference group of patients with multiple myeloma.

A level of expression of ALDH2, CTSF, FAM49A at least 1.6 times, and/or a level of expression of ATF4 at least 1.25 times higher than the average level of expression observed in the global group of subject is indicative of a higher probability of survival of the patient. Conversely, a level of expression of the genes CTSF, FAM49A, at least 2.4 times, and/or a level of expression of the genes ALDH2, ATF4 at least 1.5 times, lower than the average level of expression observed in the global group of subjects is indicative of a lower probability of survival of the patient.

A level of expression of CPSF6, STMN1, CNDP2, AFG3L2 at least 2.5 times, a level of expression of STK38, FRY, C20orf100 at least 1.5 times, and/or a level of expression ofPARP1, MGST1, LOC151162, FLJ21438 at least 1.25 times lower than the average level of expression observed in the global group of subjects is indicative of a higher probability of survival of the patient. Conversely, a level of expression of CPSF6, STK38, STMN1, CNDP2, AFG3L2, C20orf100, at least 3 times, a level of expression of LOC151162, FRY, at least 2.3 times, and/or a level of expression of PARP1, MGST1, FLJ21438 at least 1.5 times, higher than the average level of expression observed in the global group of subject is indicative of a lower probability of survival of the patient.

Methods for measuring the level of expression of a given gene are familiar to one of skill in the art. They include typically methods based on the determination of the level of transcription (i.e. the amount of mRNA produced) of said gene, and methods based on the quantification of the protein encoded by the gene.

Preferably, for carrying out the present invention, the level of expression the 15 genes listed above is based on the measurement of the level of transcription. This measurement can be performed by various methods which are known in themselves, including in particular quantitative methods involving reverse transcriptase PCR (RT-PCR), such as real-time quantitative RT-PCR (qRT-PCR), and methods involving the use of DNA arrays (macroarrays or microarrays).

Classically, methods involving quantitative RT-PCR comprise a first step wherein cDNA copies of the mRNAs obtained from the biological sample to be tested are produced using reverse transcriptase, and a second step wherein the cDNA copy of the target mRNA is selectively amplified using gene-specific primers. The quantity of PCR amplification product is measured before the PCR reaction reaches its plateau. In these conditions it is proportional to the quantity of the cDNA template (and thus to the quantity of the corresponding mRNA expressed in the sample). In qRT-PCR the quantity of PCR amplification product is monitored in real time during the PCR reaction, allowing an improved quantification (for review on RT-PCR and qRT-PCR, cf. for instance: FREEMAN et al., Biotechniques, 26, 112-22, 24-5, 1999; BUSTIN & MUELLER, Clin Sci (Lond), 109, 365-79, 2005). Parallel PCR amplification of several target sequences can be conduced simultaneously by multiplex PCR.

A "DNA array" is a solid surface (such as a nylon membrane, glass slide, or silicon or ceramic wafer) with an ordered array of spots wherein DNA fragments (which are herein designated as "probes") are attached to it; each spot corresponds to a target gene. DNA arrays can take a variety of forms, differing by the size of the solid surface bearing the spots, the size and the spacing of the spots, and by the nature of the DNA probes. Macroarrays have generally a surface of more than 10 $cm^2$ with spots of 1 mm or more; typically they contain at most a few thousand spots.

In cDNA arrays, the probes are generally PCR amplicons obtained from cDNA clones inserts (such as those used to sequence ESTs), or generated from the target gene using gene-specific primers. In oligonucleotide arrays, the probes are oligonucleotides derived from the sequences of their respective targets. Oligonucleotide length may vary from about 20 to about 80 bp. Each gene can be represented by a single oligonucleotide, or by a collection of oligonucleotides, called a probe set.

Regardless of their type, DNA arrays are used essentially in the same manner for measuring the level of transcription of target genes. mRNAs isolated from the biological sample to be tested are converted into their cDNA counterparts, which are labeled (generally with fluorochromes or with radioactivity). The labeled cDNAs are then incubated with the DNA array, in conditions allowing selective hybridization between the cDNA targets and the corresponding probes affixed to the array. After the incubation, non-hybridized cDNAs are removed by washing, and the signal produced by the labeled cDNA targets hybridized at their corresponding probe locations is measured. The intensity of this signal is proportional to the quantity of labeled cDNA hybridized to the probe, and thus to the quantity of the corresponding mRNA expressed in the sample (for review on DNA arrays, cf. for instance: BERTUCCI et al., Hum. Mol. Genet., 8, 1715-22, 1999; CHURCHILL, Nat Genet, 32 Suppl, 490-5, 2002; HELLER, Annu Rev Biomed Eng, 4, 129-53, 2002; RAMASWAMY & GOLUB, J Clin Oncol, 20, 1932-41, 2002; AFFARA, Brief Funct Genomic Proteomic, 2, 7-20, 2003; COPLAND et al., Recent Prog Horm Res, 58, 25-53, 2003)).

The CD138+ plasma cells to be analysed are classically obtained from bone marrow aspirates. Mononuclear cells are separated from the other components of the bone marrow by gradient-density centrifugation; it is recommended that separation of mononuclear cells occurs within 48 hours from the aspiration. CD138+ plasma cells are purified using immunomagnetic beads coated with an anti-CD138 antibody, or by cell sorting, to a purity > 90% assessed by morphology.

Total RNA extraction from the CD138+ plasma cells can be performed in particular by guanidinium thiocyanate-phenol-chloroform extraction using for instance TRIZOL® reagents (INVITROGEN), or by selective binding to silicagel membranes, using for instance the RNeasy® or the AllPrep® kit (QIAGEN).

The integrity of RNA for each sample is assessed for instance with the 2100 Bioanalyzer (Agilent Technologies), using the 'RNA Integrity Number' (RIN) algorithm (SCHROEDER et al., BMC Molecular Biology, 7, 3, 2006) for calculating the RNA integrity. A RIN number higher than 8 is recommended for optimal results, in particular in the case of DNA arrays. The quality and the concentration of the RNA are assessed by spectophotometry, using for instance a Nanodrop® spectrophotometer. A 260/280 ratio > 1.8, a 260/230 ratio > 1.9 and a total RNA quantity > 1 $\mu$g are recommended for optimal results.

According to a preferred embodiment of the invention, the level of transcription of the 15 genes listed above is measured, and a risk score (Pi) for a given patient is calculated according to the following equation:

$$Pi = (PARP1cr \times 0.27578783) + (CPSF6cr \times 0.26987655) + (STK38cr \times 0.29530369) + (STMN1cr \times 0.31490195) - (ALDH2cr \times 0.13137903) + (MGST1cr \times 0.17772804) + (CNDP2cr \times 0.38697337) + (AFG3L2cr \times 0.30371178) + (LOC151162cr \times 0.25043791) - (FAM49Acr \times 0.29483393) + (FLJ21438cr \times 0.19243758) - (ATF4cr \times 0.2491429) - (CTSFcr \times 0.17822457) + (FRYcr \times 0.21255699) + (C20orf100cr \times 0.21956366)$$

wherein "GENE SYMBOLcr" represents the centered value of the expression of the corresponding gene in said patient.

Said centered value is calculated according to the following equation:

$$(E - M)/SD$$

wherein E is the expression value of the patient's gene; M is the mean of the expression values of the same gene in a reference group of patients with multiple myeloma, and SD is the standard deviation of these gene expression values in the same reference group.

When a DNA array is used, the expression value for a gene is the log2 transformed intensity of the signal produced by said gene, obtained from the DNA array.

When qRT-PCR is used, the expression value for a gene is the quantity of PCR amplification product, normalized to a reference gene, such as ACTB (actin beta) or ACTG1 (actin gammal). These two genes were found by the inventors to have an invariant level of expression (i.e. the ratio (Standard Deviation/Mean) of said level of expression is <1) all the patients tested.

The inventors have calculated the mean and the standard deviation of the expression values of the 15 genes listed above, obtained with a DNA array, from a reference group of 182 patients with multiple myeloma. They have established the following equation for calculating the risk score for a given patient:

$$Pi = (E_{PARP1} - 3.82862153)/0.9500244 \times 0.27578783 + (E_{CPSF6} - (-1.65538607))/2.96334021 \times 0.26987655 + (E_{STK38} - (-3.13834963))/2.34204291 \times 0.29530369 + (E_{STMN1} - (-1.01456694))/4.83630283 \times 0.31490195 + (E_{ALDH2} - (-0.28045406))/3.387796 \times (-0.13137903) + (E_{MGST1} - (-2.51341162))/1.32820477 \times 0.17772804 + (E_{CNDP2} - (-0.78669164))/3.87155563 \times 0.38697337 + (E_{AFG3L2} - (-3.08812437))/3.26931647 \times 0.30371178 + (E_{LOC151162} - (-3.59549615))/2.07729468 \times 0.25043791 + (E_{FAM49A} - 3.95863919)/2.66392047 \times (-0.29483393) + (FLJ21438 - (-4.80774294))/1.54506377 \times 0.19243758 + (E_{ATF4} - 6.71896696) /0.79740321 \times (-0.2491429) + (E_{CTSF} - 2.7101108)/2.3864434 \times (-0.17822457) + (E_{FRY} - (-5.1706573))/2.0812732 \times (0.21255699) + (E_{C20orf100} - (-1.30853829))/3.25582089 \times (0.21956366);$$

wherein $E_{GENE}$ SYMBOL is the log2 transformed value of the expression of the corresponding gene in the tested patient.

It is believed that the estimators (mean and standard deviation) indicated in this equation are broadly usable for calculating the risk score in prospective patients, when the gene-expression values are established using a DNA array.

They are more particularly suitable for calculating the risk score in subjects which have not previously received a chemotherapy, and which are to be treated with high dose chemotherapy and autologous stem cell transplantation.

By way of example, a treatment representative of high-dose chemotherapy is the following: (i) Initial chemotherapy.with a continuous intravenous infusion of 0.4 mg of vincristine per square meter of body surface area and 9 mg of doxorubicin per square meter over a 24 hour period for 4 days, with 40 mg of oral dexamethasone per day on days 1 through 4 (the VAD regimen). Three to four cycles of VAD are administered at 3-week intervals. (ii) Autologous stem cell collection and double autologous stem cell transplantation (ASCT), melphalan alone is given before each ASCT (140 mg per square meter before the first transplant and 200 mg per square meter before the second). (iii) Maintenance, after the second ASCT, patients receive thalidomide (between 50-400 mg/day adapted according to treatment-related toxicity.

A risk score lower or equal to - 0.350 is indicative of a probability of survival at 3 years of about 95% (low-risk group); a risk score higher than -0.350 and lower or equal to + 0.820 is indicative of a probability of survival at 3 years of about 80% (intermediate-risk group). A risk score higher than + 0.820 is indicative of a probability of survival at 3 years lower than 50% (high-risk group).

The 15-gene molecular classifier of the invention can also be used in conjunction with conventional markers useful for evaluating the probability of survival of patients with multiple myeloma. In particular, it can be advantageously combined with $S\beta_2M$.

The present invention also provides kits for evaluating the probability of survival of multiple myeloma patients using the 15-gene molecular classifier of the invention.

A kit of the invention comprises a combination of reagents allowing to measure the level of expression of each of the 15 genes of said molecular classifier.

Preferably, said kit is designed to measure the level of mRNA of each of these genes. Accordingly, it comprises, for each of the 15 genes, at least one probe or primer that selectively hybridizes with the transcript of said gene, or with the complement thereof.

According to a preferred embodiment of the invention said kit comprises a DNA array. In this case said DNA array will comprise at least 15 different probes, i. e. at least one probe for each of the 15 genes of the molecular classifier.

Said probes can be cDNA probes, or oligonucleotide probes or probe sets.

A non-limitative example of a DNA array of the invention, comprising 15 cDNA probes, is more specifically described in the Examples below. One of skill in the art can easily find other suitable probes, on the basis of the sequence information available for these genes. For instance, other suitable cDNA probes can be found by querying the EST databases with the cDNA reference sequences listed in Table 1. They can also be obtained by amplification from human cDNA libraries using primers specific of the desired cDNA. Suitable oligonucleotide probes can be easily designed using available software tools (For review, cf. for instance: LI & STORMO, Bioinformatics, 17, 1067-76, 2001; EMRICH et al., Nucleic Acids Res, 31, 3746-50, 2003; ROUILLARD et al., Nucl. Acids Res., 31, 3057-62, 2003)

According to another preferred embodiment of the invention, said kit is a PCR kit, which comprises a combination of reagents, allowing specific PCR amplification of the cDNA of each of the 15 genes of the molecular classifier of the invention; these reagents include in particular at least 15 different pairs of primers i. e. at least one specific pair of primers for each of the 15 genes of the molecular classifier.

In the same way as oligonucleotide probes, suitable primers can easily be designed by one of skill in the art, and a broad variety of software tools is available for this purpose (For review, cf. for instance: BINAS, Biotechniques, 29, 988-90, 2000; ROZEN & SKALETSKY, Methods Mol Biol, 132, 365-86, 2000; GORELENKOV et al., Biotechniques, 31, 1326-30, 2001; LEE et al., Appl Bioinformatics, 5, 99-109, 2006; YAMADA et al., Nucleic Acids Res, 34, W665-9, 2006).

Optionally, said primers can be labeled with fluorescent dyes, for use in multiplex PCR assays.

The DNA arrays as well as the PCR kits of the invention may also comprise additional components, for instance, in the case of PCR kits, a pair of primers allowing the specific amplification of the reference gene ACTB and a pair of primer allowing the amplification of the reference gene ACTG1.

The invention will be further illustrated by the additional description which follows, which exemplifies the use of the molecular classifier of the invention in for predicting survival of patients with multiple myeloma. It should be understood however that this example is given only by way of illustration of the invention and does not constitute in any way a limitation thereof.

## EXAMPLE 1: SELECTION AND GROUPING OF PATIENTS

### Patients' characteristics

This study has been approved by the Institutional Ethics Committes of the Universities of Toulouse, Grenoble and Nantes and informed consent of the patients was obtained according to the Declaration of Helsinki.

Multiple myeloma patients at diagnosis with enough available bone marrow CD138+ plasma cells were identified from the files of the Hematology department at University Hospital in Nantes, France between April 2000 and October 2003 (n=250).

Bone marrow specimens from these untreated MM patients were obtained during standard diagnostic procedures in IFM centers and overnight shipped to the Hematology department at University Hospital in Nantes for further analysis.

All patients received high dose chemotherapy with stem cell transplantation according to the IFM 99 protocols. Briefly, patients received an induction therapy with 4 courses of VAD (vincristine, adriamycin and dexamethasone), followed by double intensive therapy. The IFM99-02 trial was dedicated for patients (n=186) with less than 2 poor-prognosis factors ($\beta$2-microglobulin >3 mg/1, del(13) by FISH). After induction, patients received 2 courses of high-dose melphalan (140 mg/m$^2$ and 200 mg/m$^2$), and were then randomized for maintenance therapy: none (arm A), pamidronate (arm B), or pamidronate + thalidomide (arm C) until relapse. The IFM99-03 trial enrolled patients (n=12) with 2 poor-prognosis factors and with an HLA-identical familial donor. After induction, patients received one high-dose melphalan course (200 mg/m$^2$), followed by a reduced intensity conditioned allogeneic transplant. Finally, the IFM99-04 trial enrolled patients (n=52) with 2 poor-prognosis factors and no HLA-identical familial donor. After a similar induction and first high-dose melphalan course, patients received a second melphalan-based intensification (220 mg/m$^2$), and were randomized to receive or not an anti-IL6 antibody during the conditioning regimen.

### Training and validation groups

To develop and test a predictor of survival based on gene expression, the total myeloma patients were randomly divided into two groups, the training group, and the validation group. Training-validation mode was chosen for internal validation in a 3/4-1/4 manner to obtain sufficiently large groups. Training and validation sets were stratified according to death and known confounders. The two groups included respectively 182 patients for the training set, and 68 for the validation set. Absence of significant difference between training and validation sets for baseline characteristics was verified before gene determination to avoid confounding, which could occur if the main prognostic factors were not equally distributed amongst the two sets.

No bias was observed with regard to variables examined ie S$\beta_2$M, platelets, hemoglobin, serum albumin, del13 by FISH, t(4;14), t(11;14), follow-up, survival at 3 years and ISS (Table 1).

Fifty-five patients died because of their disease during the follow-up (median= 35 months, range: 1 to 60). Characteristics of the 250 patients are shown in Table II below.

Table II

| Characteristic | Training population (n=182) | Validation population (n=68) | p-value |
|---|---|---|---|
| S$\beta$2M,mg/L | 4.6 $\pm$ 5.4* | 5 $\pm$ 4.2 | 0.56 |
| Platelets, 10$^9$/L | 261 $\pm$ 96 | 246 $\pm$ 115 | 0.36 |

(continued)

| Characteristic | Training population (n=182) | Validation population (n=68) | p-value |
|---|---|---|---|
| Hemoglobin, g/dL | 11.2 ± 6.9 | 10.4 ± 2.1 | 0.43 |
| Serum albumin, g/L | 38.8 ± 6.5 | 39.3 ± 7.8 | 0.63 |
| De113, by FISH | 89/182 | 30/68 | 0.50 |
| t(4;14) | 23/167 | 4/63 | 0.17 |
| t(11 ; 14) | 37/172 | 8/63 | 0.14 |
| Follow-up, mo | 35 ± 13 | 35 ± 15 | 0.84 |
| 3-yr survival, % | 79.7 ± 3.3 | 80.6 ± 5.3 | 0.92 |
| ISS 1<br>2<br>3 | 76<br>55<br>40 | 20<br>24<br>16 | <br><br>0.32 |
| *All such values are means ± SD | | | |

It is to be noted that the main bioclinical and cytogenetic characteristics ($S\beta_2M$; serum albumin; De113, t(4 ; 14); t(11 ; 14)) of these 250 patients do not differ from those of the rest of the patients (n=719) which were enrolled in the IFM 99 trials.

Thus, it is believed that a prognostic model established from this population of 250 patients can be broadly extrapolated to multiple myeloma patients treated according to any of the IFM 99 protocols, or similar protocols.

## EXAMPLE 2: SAMPLE COLLECTION, PLASMA CELLS PURIFICATION AND TOTAL RNA EXTRACTION AND PURIFICATION

Mononuclear cells were separated by gradient-density centrifugation (Ficoll-Hypaque, Eurobio, Les Ulis, France) from the bone marrow specimens obtained as described in Example 1.

Plasma cell purification was performed as previously described (AVET-LOISEAU et al., Blood, 99, 2185-91, 2002), on the basis of CD138 expression. Briefly, bone marrow mononuclear cells were separated using gradient density (Ficoll-Hypaque) and then incubated with anti-CD 138-coated magnetic beads (Miltenyi Biotec, Auburn, CA). Cells were passed through columns, allowing to sort plasma cells. Recovery and purity of the plasma cells were evaluated by morphology. In all cases purity of the plasma cells was higher than 90 percent assessed by morphology.

Total RNA extraction and purification were done as previously described (MAGRANGEAS et al., Blood, 101, 4998-5006, 2003), using the guanidinium thiocyanate-phenol method (CHOMCZYNSKI & SACCHI, Anal Biochem, 162, 156-9, 1987).

The purity and integrity of RNA preparations was assessed with 2100 Bioanalyzer (Agilent Technologies (Palo Alto, CA) using Agilent 2100 Expert software, the 'RNA Integrity Number' (RIN) algorithm calculated the RNA integrity for each sample. The average RIN number was 9.1 (range 6.9-10).

## EXAMPLE 3: CONSTRUCTION OF cDNA MICROARRAYS AND HYBRIDIZATION OF cDNAS DERIVED FROM MULTIPLE MYELOMA PATIENS mRNAS

### Construction of cDNA microarrays

One channel DNA microarrays were constructed from 17134 EST cDNA clones representing 11250 unique genes (based on Homo sapiens: UniGene Build #196, issued in October 2006). End-sequence-verified I.M.A.G.E. clones were purchased from RZPD German Resource Center for Genome Research (Berlin, Germany) or provided by the Human Genome Mapping Project Resource Centre (Hinxton, UK) and sequenced by MilleGen (Labege France).

The cDNA clones were amplified in 96-well microtiter plates with universal primers. PCR products were spotted onto two Hybond N+ filters GE Healthcare Life Science (Chalfont St. Giles, UK and Uppsala) using Microgrid II Biorobotics (Genomic Solutions Huntingdon, UK). The feasibility, reproducibility and sensitivity of spotting procedures onto nylon membrane currently used in our laboratory to produce cDNA arrays have been previously described (NGUYEN et al., Genomics, 29, 207-16, 1995; BERNARD et al., Nucleic Acids Res, 24, 1435-42, 1996; BERTUCCI et al., Hum. Mol. Genet., 8, 1715-22, 1999).

**Synthesis and hybridization of target cDNAs**

Target synthesis and hybridization were conducted as follows: between 0.4 and one μg of total RNA extracted from plasma cells as disclosed in Example 2, was used as template to generate cDNA bearing T7 promoter, then antisense RNA (aRNA) was generated by *in vitro* transcription using MEGAscript technology according to the Ambion protocol (Ambion Inc., Austin TX). An aliquot of 2μg of labelled aRNA was then primed with random hexaprimers and reverse transcribed with a mix of cold dNTPs and [α-33P]dCTP. Labelled cDNAs were then hybridized in 0.3 ml hybridization mix (5x SSC , 5x Denhardt's, 0.5% SDS) in scintillation vials for 48 h at 68° C. After hybridization filters were washed twice in 0.1 x SSC, 0.1 % SDS at 68° C for 90 min.

DNA microarrays were scanned at 25-μm resolution using a Fuji BAS 5000 image plate system (Raytest, Paris, France). The hybridization signals were quantified using ArrayGauge software v.1.3 (Fuji, Ltd, Tokyo, Japan). For each membrane, the data were normalized by the global intensity hybridization. A background value was calculated from negative controls $\pm$ 6 SD and subtracted to each value. After expression data correction for the amount of PCR product spotted onto the membrane, 7 508 features detected in at least 5% of the patients were retained for subsequent analysis.

**EXAMPLE 4: SELECTION OF GENES SIGNIFICANTLY ASSOCIATED WITH SURVIVAL**

SAS System version 9.1 (SAS Institute Inc., Cary, NC) and BRB-ArrayTools software developed by Dr. Richard Simon and Amy Peng, version 3.4.0 (Simon et a]., 2003; available at http://linus.nci.nih.gov/BRB-ArrayTools.html) were used to perform statistical analyses.

Principal aim was maximal reduction of gene set size with minimal loss of prognostic information. Raw intensities of the microarray data were transformed into $\log_2$ intensities before proceeding with univariate Cox analysis. Univariate Cox analyses were conducted on the 7508 gene probes. As usual in microarrays data analysis great stringency (p-value<0.001) was needed to establish criteria for gene selection, giving a 50-gene list. Then, in order to maximize reduction of overfitting, resampling (n=1000, 80-20%) and permutation (n=1000) were used in the training set. This confirmed p-values <0.005 and <0.005 respectively for 28 genes from the 50-gene list. At last to verify stability of this 28-gene list, we determined a survival predictors (high risk vs. low risk) by means of BRB-Arrays Tool for each of 100 random training/test sets. The 100 predictors gene lists were intersected and only 15 genes which were present in at least 50% of the predictors (mean:75% - range: 56% to 97%) were kept. All these genes had individual false discovery rate (FDR)<1.5% (mean: 0.9% - range: 0.0001 % to 1.4%).

These genes are listed in Table III below.

TABLE III

| UMGC probe | Clone IMAGE | GenBank Acc | Reference Sequence | Gene symbol | Cytoband |
|---|---|---|---|---|---|
| UMGC 06566 | 470455 | AA031267 | NM_018235 | CNDP2 | 18q22.3 |
| UMGC 01066 | 53227 | R15906 | AK123488 | STMN1 | 1p36.1-p35 |
| UMGC 06118 | 156875 | R74045 | NM_006796 | AFG3L2 | 18p11 |
| UMGC 05764 | 34211 | R20152 | NM_007271 | STK38 | 6p21 |
| UMGC 01969 | 795472 | AA454196 | NM_001618 | PARP1 | 1q41-q42 |
| UMGC 08943 | 264153 | BX094475 | NM_007007 | CPSF6 | 12q15 |
| UMGC 00460 | 2013483 | A1375333 | BX647087 | LOC151162 | 2q21.3 |
| UMGC 11582 | 41332 | BX098850 | NM_032883 | C20orf100 | 20q13.12 |
| UMGC 11580 | 44515 | BX095181 | NM_023037 | FRY | 13q13.1 |
| UMGC 10992 | 2254655 | A1801844 | AK024488 | FLJ21438 | 19p13.12 |
| UMGC_07324 | 110238 | T71465 | NM_145791 | MGST1 | 12p12.3-p12.1 |
| UMGC 02996 | 125721 | R07565 | NM_000690 | ALDH2 | 12q24.2 |
| UMGC 09916 | 739205 | AA421327 | NM_003793 | CTSF | 11q13 |
| UMGC 11702 | 46141 | H09039 | NM_001675 | ATF4 | 22q13.1 |
| UMGC 17217 | 28465 | R13378 | AK055334 | FAM49A | 2p24.3 |

This table indicates the internal (UMGC) reference of the cDNA probe spotted on the array, the reference of the IMAGE clone from which this cDNA probe was obtained, the GenBank Accession N° corresponding to the partial sequence of the insert of this clone, the Reference Sequence, corresponding to the representative mRNA sequence, the HGNC Gene symbol of the corresponding gene, and the localization on this gene on the human chromosome.

## EXEMPLE 5: CONSTRUCTION OF A 15-GENE SURVIVAL CLASSIFIER AND CALCULATION OF A RISK SCORE BASED ON THIS CLASSIFIER

Principal component analysis (PCA) was performed to summarize with minimal loss the 15-gene list information. PCA is a multivariate technique that permits reduction of dimensionality and detection of linear relationships. Table IV below indicates the PCA score for each of the 15 genes.

Table IV

| UMGC probe | Gene symbol | PCA score |
|---|---|---|
| UMGC_06566 | CNDP2 | 0.39 |
| UMGC 01066 | STMN1 | 0.31 |
| UMGC 06118 | AFG3L2 | 0.30 |
| UMGC 05764 | STK38 | 0.30 |
| UMGC 01969 | PARP1 | 0.28 |
| UMGC 08943 | CPSF6 | 0.27 |
| UMGC_00460 | LOC151162 | 0.26 |
| UMGC 11582 | C20orf100 | 0.22 |
| UMGC 11580 | FRY | 0.21 |
| UMGC 10992 | FLJ21438 | 0.19 |
| UMGC 07324 | MGST1 | 0.18 |
| UMGC 02996 | ALDH2 | -0.13 |
| UMGC_09916 | CTSF | -0.18 |
| UMGC 11702 | ATF4 | -0.25 |
| UMGC 17217 | FAM49A | -0.29 |

The first principal component (the one with the largest variance of any linear combination of these genes), was used to calculate an expression risk score according to the following formula:

$$\text{Risk score} = (UMGC\_01969cr \times 0.27578783) + (UMGC\_08943cr \times 0.26987655) + (UMGC\_05764cr \times 0.29530369) + (UMGC\_01066cr \times 0.31490195) - (UMGC\_2996cr \times 0.13137903) + (UMGC\_07324cr \times 0.17772804) + (UMGC\_06566crx 0.38697337) + (UMGC\_06118cr \times 0.30371178) + (UMGC\_00460cr \times 0.25043791) - (UMGC\_017217cr \times 0.29483393) + (UMGC\_10992cr \times 0.19243758) - (UMGC\_11702cr \times 0.2491429) - (UMGC\_09916cr \times 0.17822457) + (UMGC\_11580cr \times 0.21255699) + (UMGC\_11582cr \times 0.21956366).$$

"UMGC_####" cr represented the centered value of each particular probe.
Using, for each of the 15 genes, the mean and the standard deviation of its expression value, calculated from the 182 patients of the training set, the following equation was obtained:

$$\text{Risk score} = (UMGC\_01969-3.82862153)/0.9500244*0.27578783 + (UMGC\_08943-(-1.65538607)) /2.96334021*0.26987655 + (UMGC\_05764-(-3.13834963))/2.34204291*0.29530369 + (UMGC\_01066-(-1.01456694)) /4.83630283*0.31490195 + (UMGC\_02996-(-0.28045406)) /3.387796*(-0.13137903) + (UMGC\_07324-(-2.51341162)) /1.32820477*0.17772804 + (UMGC\_06566-(-0.78669164)) /3.87155563*0.38697337 + (UMGC\_06118-(-3.08812437)) /3.26931647*0.30371178 + (UMGC\_00460-(-3.59549615)) /2.07729468*0.25043791 + (UMGC\_17217-3.95863919) /2.66392047*(-0.29483393) + (UMGC\_10992-(-4.80774294)) /1.54506377*0.19243758 + (UMGC\_11702-6.71896696) /0.79740321*(-0.2491429) + (UMGC\_09916-2.7101108) /2.3864434*(-0.17822457) + (UMGC\_11580-(-5.1706573)) /2.0812732*(0.21255699) + (UMGC\_11582-(-1.30853829)) /3.25582089*(0.21956366)$$

This equation was used to calculate a risk score for each patient in the training group. Patients were ranked according to this score and divided into quartiles. The same procedure was used to calculate a risk score for each patient in the validation group. These patients were then classified into risk groups according cut off values calculated form the training group only.

Since quartiles 1 and 2 were not different for overall survival (p=0.727) they were pooled into low-risk group, quartile 3 and quartile 4 were called respectively intermediate-risk and high-risk groups.

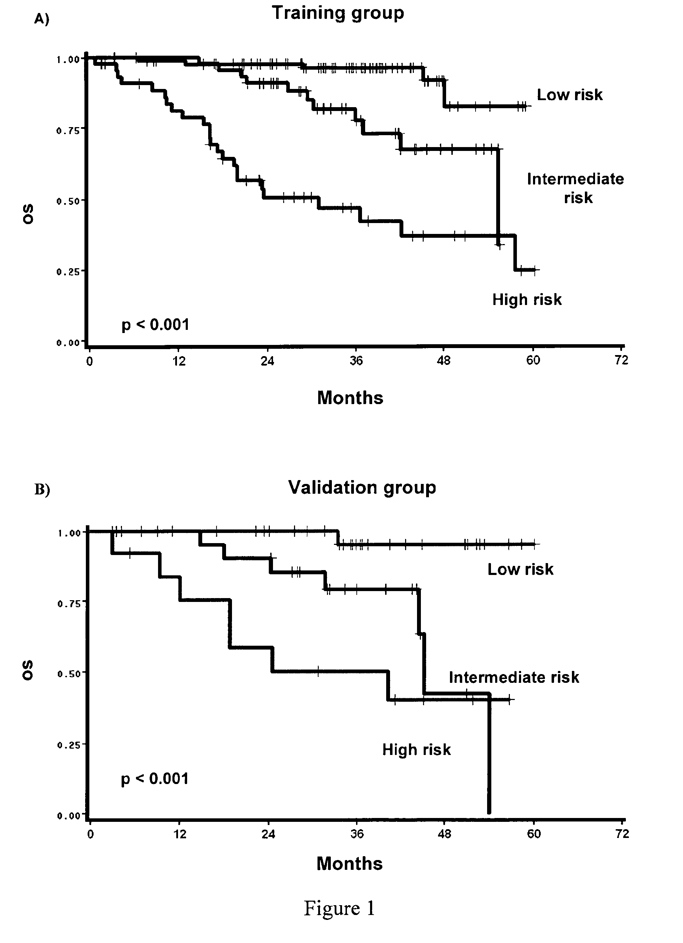

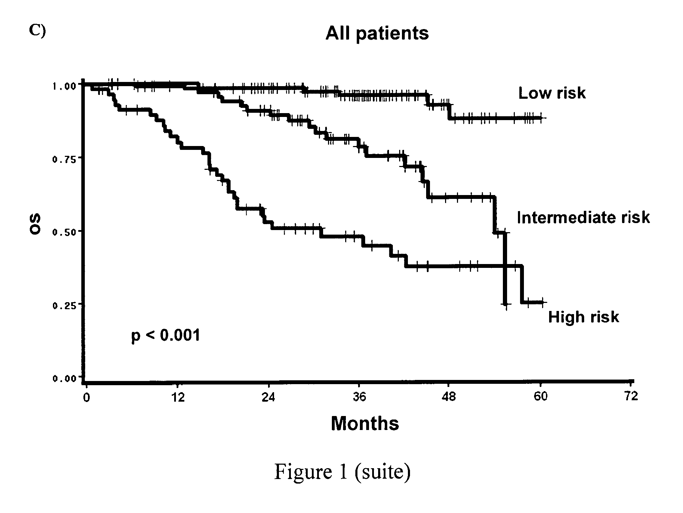

Figure 1 shows the Kaplan-Meier analysis of overall survival (OS) among myeloma patients in the training group (A), the validation group (B), and all patients (C).

These curves clearly show differences among patients stratified as having a low, intermediate or high risk by the 15-gene classifier score.

Table V below shows the Kaplan-Meier estimates of the rate of survival at 3 years, according to 15-gene classifier categories; the proportions of patients who survived at 3 years were, 95.1 percent, 81.3 percent and 47.4 percent, respectively.

TABLE V

| Risk category | Number of patients | Rate of survival at 3 Yr (95% CI)* percent |
|---|---|---|
| Low | 125 | 95.1(88.4-97.9) |
| Intermediate high | 63 | 81.3 (66.5 - 90.1) |
| High | 62 | 47.4(33.5-60.1) |
| *CI denotes confidence interval | | |

Thus, the 15-gene classifier was highly predictive of survival in the training group (p<0.001) and in the test group (p<0.001), Kaplan-Meier curves of overall survival clearly showed differences among patients stratified as having a low, intermediate or high risk by the 15-gene classifier score (Fig.1),

**EXAMPLE 6: COMPARISON OF THE 15-GENE CLASSIFIER WITH KNOWN PROGNOSTIC VARIABLES**

Univariate and multivariate analyses were performed on the whole cohort to determine the relative prognostic values of known prognostic variables and the 15-gene classifier. In addition bootstrap and permutation techniques were used to assess the significance of the variables.

Univariate logrank analysis was performed for each bioclinical variable and the 15-gene classifier in the original data set. In order to avoid overfitting, bootstrap and permutation techniques were used to assess the statistical significance obtained with the original data sets. First, each bioclinical parameter was tested upon the 1000 bootstrap samples randomly created and the number of bootstrap p-values lower than 0.05 was counted. This process was then also applied to 1000 permuted samples randomly created. Only parameters with at least 500 bootstrapped p-values<0.05 and with permutation p-value<0.05 were retained in multivariate analysis.

The results are shown in Table VI below.

Table VI

| Variable | Original data set Log rank p-value | Bootstrap(n=1000) Log rank p-value <0.05 | Permutation (n=1000) Log rank p-value |
|---|---|---|---|
| $S\beta_2M \geq$ 5.5 mg/L | < 0.001 | 900 | 0.011 |
| Serum albumin < 30 g/L | 0.013 | 676 | 0.042 |
| Platelets <130 $10^9$/L | < 0.001 | 938 | 0.004 |
| Del13, by FISH | 0.006 | 814 | 0.001 |
| t(4;14) | 0.003 | 789 | 0.030 |
| 15-gene classifier | < 0.001 | 1000 | < 0.001 |

These results show that the 15-gene classifier performs significantly better (p<0.001) than the 5 variables significantly associated to survival (p< 0.05) ie $S\beta_2M \geq$ 5.5 mg/L, serum albumin < 30 g/L, platelets <130 $10^9$/L, t(4;14) and del13 by FISH.

Multivariate Cox proportional-hazards analysis was performed to evaluate the relation between overall survival and the variables significant in the univariate analysis. In order to strengthen results obtained from original data set and to obtain robust estimates, permutation and boostrap techniques were also applied and results were compared to the original data set.

The results are shown in Table VII below

Table VII

| Factor | Hazard Ratio | 95% HR CI* | p-value |
|---|---|---|---|
| $S\beta_2M \geq$ 5.5 mg/L | 2.55 | 1.09 - 5.96 | 0.030 |
| Serum albumin < 30 g/L | 1.09 | 0.42 - 2.85 | 0.866 |
| Platelets <130 $10^9$/L | 2.21 | 0.87-5.61 | 0.096 |
| Del 13, by FISH | 0.99 | 0.44 - 2.29 | 0.998 |
| t(4;14) | 1.91 | 0.74 - 4.90 | 0.181 |
| 15-gene classifier risk category: Intermediate<br>High | 4.42<br>10.18 | 1.50 - 12.98<br>3.34 - 31.04 | 0.007<br><0.001 |
| * CI denotes confidence interval | | | |

The model retained only two variables independently associated with the prognosis: 15-gene classifier (intermediate-risk or high-risk vs low-risk) and $S\beta_2M$ ($\geq$ 5.5 mg/L vs < 5.5mg/L).

The 15-gene survival classifier was by far the most powerful prognostic factor, with a hazard ratio of 4.4 (95 percent confidence interval, 1.5 to 13) in the intermediate-risk group and a hazard ratio of 10.2 (95 percent confidence interval, 3.3 to 31) in the high-risk group. Not surprisingly the other variable retained in the model was $S\beta_2M \geq$ 5.5 mg/L, since high $S\beta_2M$ value was recently confirmed as the strongest clinical prognostic variable that delineated high-risk group (ISS 3) by the ISS system (GREIPP et al., J Clin Oncol, 23, 3412-20, 2005). Permutation test showed a better Akaike information criterion than for original data only 3 times (p=0.003) and bootstrap procedure repeated 1000 times provided hazard ratios close to values from original data set thus demonstrating the robustness of the estimates (data not shown).

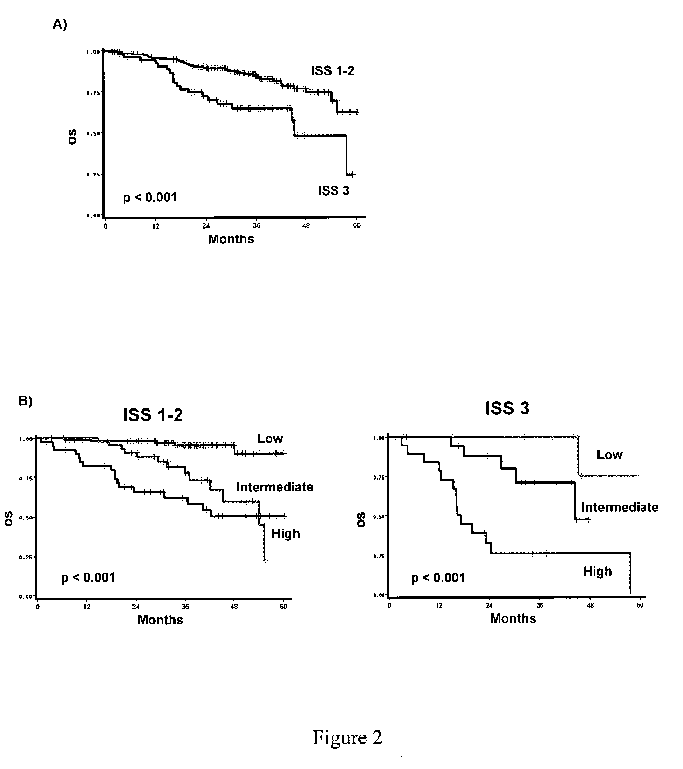

Kaplan Meier curves of overall survival among patients in the high-risk group (ISS 3, $S\beta_2M \geq$ 5.5 mg/L), $S\beta_2M \geq$ 5.5 mg/L and in the low/intermediate-risk group (ISS 1-2, $S\beta_2M$ < 5.5 mg/L) according to the international staging system are shown in Figure 2A. Kaplan Meier curves of overall survival among patients for the indicated ISS risk groups categorized according to the 15-gene classifier risk score are shown in Figure 2B.

These Kaplan Meier curves show the independence of the ISS and the 15-gene survival classifier (Fig 2). Among patients predicted to be low/intermediate-risk (ISS I-2) or high-risk (ISS 3 or $S\beta_2M \geq$ 5.5 mg/L) scores (Fig. 2A), the 15-gene classifier dissected these two subsets of patients into 3 risk groups with significantly different survivals (Fig 2B). These results indicate that 15-gene classifier and ISS marked distinct biological features associated with survival. Of particular interest the 15-gene survival classifier score identified the highest risk patients in the ISS 3 group, with a median survival of 17 months (Fig 2B).

Combining 15-gene survival classifier score and $S\beta_2M$ yielded a powerful predictive model with 4 risk groups: 15-gene classifier low (RG0), 15-gene classifier intermediate (RG1), 15-gene classifier high and $S\beta_2M < 5.5$ mg/L (RG2), 15-gene classifier high and $S\beta_2M \geq 5.5$ mg/L (RG3).

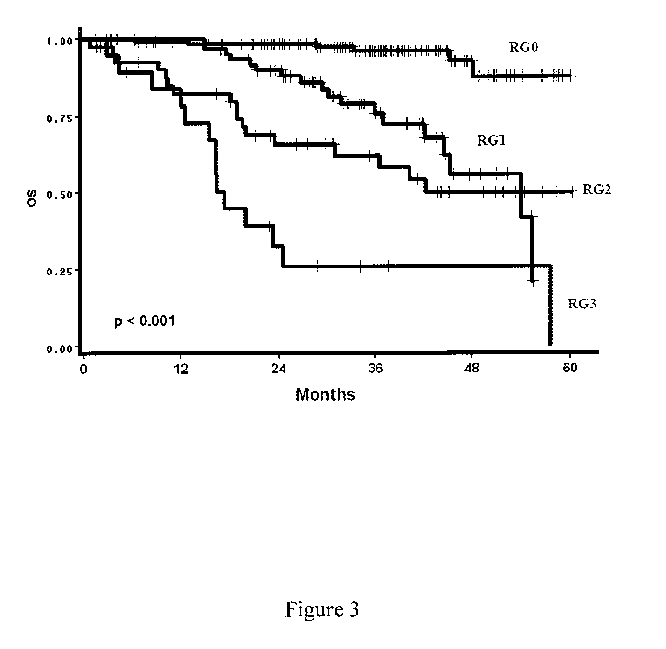

The distribution of patients into these 4 categories and hazard ratio are shown in Table VIII below, and the results of the Kaplan Meier analysis are shown in Figure 3.

Table VIII

| Risk Group (RG) IS Group (RG) | Distribution of patients, % | 3-year OS, % (95% CI)* | Hazard ratio (95% CI) |
|---|---|---|---|
| 15-gene classifier Low (RG0) | 125(50%) 125 (50%) | 95.1 1 (88.4-97.9) | |
| 15-gene classifier Intermediate (RG1) | 62 (25%) 62(25%) | 81.2 (66.3-89.9) | 4.3 (1.7-10.7) |
| 15-gene classifier High + Sβ2M < 5.5 mg/L (RG2) | 42 (17%) | 57.1 (39.3 - 71.3) | 10.5 (4.4-24.8) |
| 15-gene classifier High + Sβ2M ≥ 5.5 mg/L (RG3) | 20 (8%) | 26.1 (8.6-47.9) | 26.5 (10.6-66.5) |
| *CI denotes confidence interval | | | |

This model showed a highly predictive power. Half of the patients (R0) were predicted as having a risk of death at 3 years less than 5 percent while each progression from R1 to R3 was associated with an increase in the hazard ratio of death by a factor of approximately 2.5. The Kaplan Meier analysis (Fig 3) also showed clear differences in survival according to RG classification among myeloma patients.

**Claims**

1. A method for evaluating the probability of survival at a predetermined date for a patient with multiple myeloma, said method being **characterized in that** it comprises measuring the level of expression of each of the following genes: CNDP2; STMN1; AFG3L2; STK38; PARP1; CPSF6; LOC151162; C20orf100; FRY; FLJ21438; MGST1; ALDH2; CTSF; ATF4; FAM49A, in a sample of bone marrow CD138+ plasma cells obtained from said patient.

2. The method of claim 1, wherein the level of expression of said genes is measured by determination of their level of transcription, using a DNA array.

3. The method of claim 1, wherein the level of expression of said genes is measured by determination of their level of transcription, using quantitative RT-PCR.

4. The method of any of claims 1 to 3, wherein and a risk score (Pi) for a given patient is calculated according to the following equation:

$$Pi = (PARP1cr \times 0.27578783) + (CPSF6cr \times 0.26987655) + (STK38cr \times 0.29530369) + (STMN1cr \times 0.31490195) - (ALDH2cr \times 0.13137903) + (MGST1cr \times 0.17772804) + (CNDP2cr \times 0.38697337) + (AFG3L2cr \times 0.30371178) + (LOC151162cr \times 0.25043791) - (FAM49Acr \times 0.29483393) + (FLJ21438cr \times 0.19243758) - (ATF4cr \times 0.2491429) - (CTSFcr \times 0.17822457) + (FRYcr \times 0.21255699) + (C20orf100cr \times 0.21956366)$$

wherein "GENE SYMBOLcr" represents the centered value of the level of expression of the corresponding gene in said patient.

5. The method of claim 3, wherein a risk score (Pi) for a given patient is calculated according to the following equation:

$$Pi = (E_{PARP1} - 3.82862153)/0.9500244 \times 0.27578783 + (E_{CPSF6} - (-1.65538607))/2.96334021 \times 0.26987655 + (E_{STK38} - (-3.13834963))/2.34204291 \times 0.29530369 + (E_{STMN1} - (-1.01456694))/4.83630283 \times 0.31490195 + (E_{ALDH2} - (-0.28045406))/3.387796 \times (-0.13137903) + (E_{MGST1} - (-2.51341162))/1.32820477 \times 0.17772804 + (E_{CNDP2} - (-0.78669164))/3.87155563 \times 0.38697337 + (E_{AFG3L2} - (-3.08812437))/3.26931647 \times 0.30371178 + (E_{LOC151162} - (-3.59549615))/2.07729468 \times 0.25043791 + (E_{FAM49A} - 3.95863919)/2.66392047 \times (-0.29483393) + (FLJ21438 - (-4.80774294))/1.54506377 \times 0.19243758 + (E_{ATF4} - 6.71896696)/0.79740321 \times (-0.2491429) + (E_{CTSF} - 2.7101108)/2.3864434 \times (-0.17822457) + (E_{FRY} - (-5.1706573))/2.0812732 \times (0.21255699) + (E_{C20orf100} - (-1.30853829))/3.25582089 \times (0.21956366);$$

wherein $E_{GENE}$ SYMBOL is the log2 transformed value of the expression of the corresponding gene in the tested patient.

6. The method of any of claims 1 to 5, which further comprises measuring the level of β2-microglobulin in a sample of serum from said patient.

7. A kit for evaluating the probability of survival of multiple myeloma in a patient, **characterized in that** it comprises a combination of reagents for measuring the level of expression of each of the 15 genes listed in claim 1.

8. A kit of claim 7, **characterized in that** it contains a DNA array comprising for each of the 15 genes listed in claim 1, at least at least one nucleic acid probe specific of said gene.

9. A kit of claim 7, **characterized in that** it is a PCR kit, containing, for each of the 15 genes listed in claim 1, at least one pair of PCR primers specific of said gene.

**A)**

## Training group

**Months**

**B)**

## Validation group

**Months**

Figure 1

C)

**All patients**

Figure 1 (suite)

A)

B)

Figure 2

Figure 3

**European Patent Office**

## EUROPEAN SEARCH REPORT

Application Number

EP 07 29 0251

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHAUGHNESSY JOHN D ET AL: "A validated gene expression model of high-risk multiple myeloma is defined by deregulated expression of genes mapping to chromosome 1" BLOOD, vol. 109, no. 6, 14 November 2006 (2006-11-14), pages 2276-2284, XP002444355 ISSN: 0006-4971 Published online ahead of print. * abstract; tables 1-3,7 * ----- | 1-9 | INV. C12Q1/68 |
| X | ZHAN FENGHUANG ET AL: "The molecular classification of multiple myeloma" BLOOD, vol. 108, no. 6, September 2006 (2006-09), pages 2020-2028, XP002444356 ISSN: 0006-4971 * the whole document * ----- | 1-9 | |
| A | TASSONE ET AL: "Genetics and molecular profiling of multiple myeloma: Novel tools for clinical management?" EUROPEAN JOURNAL OF CANCER, PERGAMON PRESS, OXFORD, GB, vol. 42, no. 11, July 2006 (2006-07), pages 1530-1538, XP005584789 ISSN: 0959-8049 * the whole document * ----- -/-- | 1-9 | TECHNICAL FIELDS SEARCHED (IPC) C12Q |

The present search report has been drawn up for all claims

| Place of search | Date of completion of the search | Examiner |
|---|---|---|
| Munich | 26 July 2007 | Werner, Andreas |

EPO FORM 1503 03.82 (P04C01)

**European Patent Office**

**EUROPEAN SEARCH REPORT**

Application Number

EP 07 29 0251

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BATAILLE REGIS ET AL: "The phenotype of normal, reactive and malignant plasma cells. Identification of "many and multiple myelomas" and of new targets for myeloma therapy" HAEMATOLOGICA-THE HEMATOLOGY JOURNAL, vol. 91, no. 9, September 2006 (2006-09), pages 1234-1240, XP002444357 ISSN: 0390-6078(print) 1592-8721(ele * the whole document * | 1-9 | |
| A | MAGRANGEAS FLORENCE ET AL: "Gene expression profiling of multiple myeloma reveals molecular portraits in relation to the pathogenesis of the disease." BLOOD, vol. 101, no. 12, 15 June 2003 (2003-06-15), pages 4998-5006, XP002444358 ISSN: 0006-4971 * the whole document * | 1-9 | |
| A | SHIPP M A ET AL: "Diffuse Large B-cell Lymphoma Outcome Prediction by Gene-expression Profiling and Supervised Machine Learning" NATURE MEDICINE, NATURE PUBLISHING GROUP, NEW YORK, NY, US, vol. 8, no. 1, January 2002 (2002-01), pages 68-74, XP002990032 ISSN: 1078-8956 * the whole document * | 1-9 | TECHNICAL FIELDS SEARCHED (IPC) |

-/--

The present search report has been drawn up for all claims

| Place of search | Date of completion of the search | Examiner |
|---|---|---|
| Munich | 26 July 2007 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 07 29 0251

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | AVET-LOISEAU HERVE ET AL: "Genetic abnormalities and survival in multiple myeloma: the experience of the Intergroupe Francophone du Myelome" BLOOD, vol. 109, no. 8, April 2007 (2007-04), pages 3489-3495, XP002444359 ISSN: 0006-4971 * the whole document * | 1-9 | |
| | ----- | | |
| | | | **TECHNICAL FIELDS SEARCHED (IPC)** |

The present search report has been drawn up for all claims

| Place of search | Date of completion of the search | Examiner |
|---|---|---|
| Munich | 26 July 2007 | Werner, Andreas |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GREIPP et al.** *J Clin Oncol,* 2005, vol. 23, 3412-20
- **FREEMAN et al.** *Biotechniques,* 1999, vol. 26 (112-22), 24-5
- **BUSTIN ; MUELLER.** *Clin Sci (Lond,* 2005, vol. 109, 365-79
- **BERTUCCI et al.** *Hum. Mol. Genet,* 1999, vol. 8, 1715-22
- **CHURCHILL.** *Nat Genet,* 2002, vol. 32, 490-5
- **HELLER.** *Annu Rev Biomed Eng,* 2002, vol. 4, 129-53
- **RAMASWAMY ; GOLUB.** *J Clin Oncol,* 2002, vol. 20, 1932-41
- **AFFARA.** *Brief Funct Genomic Proteomic,* 2003, vol. 2, 7-20
- **COPLAND et al.** *Recent Prog Horm Res,* 2003, vol. 58, 25-53
- **SCHROEDER et al.** *BMC Molecular Biology,* 2006, vol. 7, 3
- **LI ; STORMO.** *Bioinformatics,* 2001, vol. 17, 1067-76
- **EMRICH et al.** *Nucleic Acids Res,* 2003, vol. 31, 3746-50
- **ROUILLARD et al.** *Nucl. Acids Res,* 2003, vol. 31, 3057-62
- **BINAS.** *Biotechniques,* 2000, vol. 29, 988-90
- **ROZEN ; SKALETSKY.** *Methods Mol Biol,* 2000, vol. 132, 365-86
- **GORELENKOV et al.** *Biotechniques,* 2001, vol. 31, 1326-30
- **LEE et al.** *Appl Bioinformatics,* 2006, vol. 5, 99-109
- **YAMADA et al.** *Nucleic Acids Res,* 2006, vol. 34, W665-9
- **AVET-LOISEAU et al.** *Blood,* 2002, vol. 99, 2185-91
- **MAGRANGEAS et al.** *Blood,* 2003, vol. 101, 4998-5006
- **CHOMCZYNSKI ; SACCHI.** *Anal Biochem,* 1987, vol. 162, 156-9
- **NGUYEN et al.** *Genomics,* 1995, vol. 29, 207-16
- **BERNARD et al.** *Nucleic Acids Res,* 1996, vol. 24, 1435-42